# EUROPEAN PATENT APPLICATION

(11) **EP 1 889 587 A2**
(43) Date of publication of application: **20.02.2008**
(21) Application number: 07019647.2
(22) Date of filing: 16.08.2002
(51) Int. Cl.: A61F 2/06

(54) **Intersomatic cage for posterior fusion surgery to the lumbar column and for surgery involving the insertion of a transforaminal implant**

(30) Priority: 16.08.2001 ES 200101905
(62) Divisional of application: 02018509.6
(71) Applicant: Biomet Spain Orthopaedics S.L., Paterna (Valencia) (ES)
(72) Inventor: Lopez Casquero, Carlos, 46988 Fuente del Jarro (ES); Sierra Aparici, Alfredo, 46988 Fuente del Jarro (ES)
(74) Representative: Koepe, Gerd L.

(57) **Abstract**

Intersomatic cage for posterior fusion surgery of the lumbar column and a transforaminal implant introducer, formed by a cage (1, 8, 16) with the aim of restoring the height of the intersomatic space between lumbar vertebrae (14) with lateral windows (2) which allow the radiological control of bone growth and a vertical trough (3), which shape an interior cavity where the bone graft (5) is housed for the fusion or arthrodesis, with teeth in the shape of a saw (6) on the upper and lower part to increase the capacity for subjection and area of support between the adjacent vertebrae. Described is also the introducer (17) made up of a handle (18), a bent tube (19) through which a flexible rod (20) passes.

## Description

### Object of the invention

This invention refers to an intersomatic cage for posterior fusion surgery to the lumbar column and for surgery involving the insertion of a transforaminal implant, which represents solutions in terms of improving the vertebral fusion of the lumbar column within the scope of the surgical procedures referred to as PLIF (Posterior Lumbar Interbody Fusion) or TLIF (Transforaminal Lumbar Interbody Fusion), by means of:
I) A monobloc intersomatic cage;
II) an expansive intersomatic cage;
III) a transforaminal intersomatic cage.

The function of these intersomatic cages is as an element acting as a spacer between and holding the lumbar vertebrae between which they are placed, in such a way that they prevent their collapse and increase the initial mechanical rigidity during lumbar fusion. At the same time, these intersomatic cages serve as a support and structural improvement in combination with other types of instrumentation, such as bars and plates joined to transpedicular screws or laminar and pedicular hooks.

### Background to the invention

The Posterior Intersomatic Fusion of the Lumbar Column or, as it is better known, Posterior Lumbar Interbody Fusion (PLIF), was initially developed by Cloward in the 1940s to treat lumbar pain due to intervertebral hernias or the degeneration of disks. In this surgical technique, the central portion of the disk is removed and replaced by multiple blocks of osseous graft from the patient himself or by artificial bone. More recently, pedicular screws and plats have been used to treat the same degenerative conditions. In 1988, Dr. Steffe recommended the use of these systems of fixing using pedicular screws in combination with these grafts, with the aim of better distributing the loads with the anterior part of the lumbar column. Nevertheless, problems have still arisen with the transplanted bone, such as the collapse of the graft due to the insufficient initial mechanical resistance and due to biological incompatibility in cases in which artificial bone had been grafted. Currently, other variants of posterior approach have been developed, such as the transforaminal route (TLIF), in which a posterolateral surgical approach is made along a transforaminal route, but which is basically the same technique as the PLIF.

In this day and age, the intersomatic cages, as they are described in this invention, resolve a high percentage of diseases related to the dorsolumbar vertebral disks. The principal surgical indications for these are:
- Damage to the disks due to traumatisms, tumours or degeneration of the vertebrae, for which decompression is necessary, as is restoring the original height of the intervertebral space by means of arthrodesis (osseous fusion).
- Chronic lumbago related to the vertebral disks, for which all the conservative treatments such as stretching exercises, analgesics, muscular relaxants etc., do not give positive results.
- It is used as an adjunct in transpedicular surgery and surgery with bars or plates, thereby increasing the rigidity of the system implanted.

The problems that are currently being created, and that are in part still not resolved for the majority of implants, are as follows:
- Incomplete fusion due in part to the actual design of the implant.
- Displacement of the dura mater with the consequent risk of neurological lesions, again due to the design of the implant and its instrumentation.
- Radiopaque materials and massive designs that do not permit verification, during the post-operative period, of whether a correct osseous fusion exists.

### Description of the invention

With the intersomatic cage for posterior fusion surgery to the lumbar column and for surgery involving the insertion of an implant, which is the object of this invention, it is sought to avoid or to palliate all of these difficulties. For this purpose, these cages are designed with the aim of providing initial mechanical resistance prior to fusion, of facilitating fusion through osseous growth through the windows that it disposes of in its design, into which autologous graft (graft from the patient himself) is inserted, and finally, of facilitating the post-operative follow-up of osseous growth through the windows or holes provided for in its design and/or use of radiotransparent materials (materials that permit the osseous growth to be scanned by X-rays), such as for example: PEEK (polyether ether ketone), Carbon Fibre or else PEEK-based Composites of Carbon Fibre.

The intersomatic cages for lumbar fusion surgery to which the invention refers are based on a system of essentially hollow intersomatic cages that come in three variants, all of which perform the same function and which are described in further detail below.

### MONOBLOCK CAGE

This monoblock cage is an intersomatic separating device for lumbar column vertebrae. It is used to restore the natural height of the disc and is used as a bridge for bone fusion. It is composed of a cage with two side windows and one vertical window that form the hollow space required to hold the bone graft that will form the fusion by growing the bone between the adjoining vertebrae. It is an essentially oval-shaped section, thus optimising the implant's contact with the bone so that it adapts to the discs in the sub-chondral zone of the vertebrae to be fused. Its distal part is also higher to maintain the natural curvature or lordosis. The distal part is rounded to facilitate its implantation without damaging nerve roots or soft spots. The top and bottom of the cage are equipped with a tilted sawtooth section with the aim to: 1) prevent the device from being expelled by a harpoon or blocking effect; 2) prevent the vertebrae from collapsing as a result of being crushed by increasing the surface area of contact with the bone; and 3) preventing any movement which impedes the proper growth of the bone between the cage and the adjacent vertebrae.

### EXPANSION CAGE

As in the case of the monoblock cage described above, the second type consists of an intersomatic separating device for lumbar column vertebrae. The applications and advantages are the same, however, the aim of this invention is to augment the fastening power of the device to the intersomatic space while maintaining the curvature or lordosis of the lumbar column after the cage expands. Its principal advantage over the other designs is the ease with which it is implanted, since the height of the distal part of the cage is initially lower before it expands once implanted, thus making implantation less traumatic.

It has a sliding washer inside which, in order to be applied, once the cage is in position, an instrument is used to push the washer against the cage, causing the distal end to expand due to the tilt of the lateral window, which serves an angulated lateral guide. The lateral window is also equipped with a stopper or projection that limits the washer's movement and prevents it from leaving the cage.

### TRANSFORAMINAL CAGE

This Transforaminal Lumbar Interbody Fusion intersomatic cage is in the shape of a circumferential arch, hollow on both sides and with orifices or threads at either end to facilitate introduction, and is equipped with a central column or nerve to make the cage more rigid. The cage is shaped in the form of a wedge, seen from the side, in order to maintain a fixed angle of lordosis in the vertebrae.

This cage offers an improvement over the previous two in that only one piece is required per implant, rather than two, with a single access channel, thus cutting down on surgical expenses and simplifying the procedure which in turn reduces the risk of damage to the dura mater.

This invention also involves a device for introducing the cage through transforaminal channels between two lumbar vertebrae consisting of a bent tube with a handle on one end, the inside of which contains a flexible threaded rod that fastens the implant (intersomatic cage).

### Description of the Drawings

To supplement the description provided herein, and for a better understanding of the characteristics of the invention, this description is accompanied by a series of figures which represents the following, on an illustrative but non-limitative basis:
Figure 1 represents a side view of the final assembly of a practical execution of the monoblock intersomatic cage to which the invention refers, relative to the placement of the cage in the intersomatic channel.
Figure 2 represents a rear view of the same assembly, relative to the placement of two intersomatic cages and bone graft from a rear view.
Figure 3 shows a view of the monoblock intersomatic cage from an isometric perspective.
Figure 4 shows a rear view of the monoblock intersomatic cage.
Figure 5 shows a side view of the monoblock intersomatic cage.
Figure 6 shows an overhead view of the monoblock intersomatic cage.
Figure 7 represents a side view of the final assembly of a practical execution of the expansive intersomatic cage to which the invention refers, relative to the placement of the cage in the intersomatic channel.
Figure 8 represents a rear view of the same assembly, relative to the placement of two expansive intersomatic cages and bone graft from a rear view.
Figure 9 shows a view of the expansive intersomatic cage from an isometric perspective.
Figure 10 shows a rear view of the expansive intersomatic cage.
Figure 11 shows a side view of the expansive intersomatic cage.
Figure 12 shows a view from an isometric perspective of washer in the expansive intersomatic cage.
Figure 13 shows an overhead view of the expansive intersomatic cage
Figure 14 shows a view of the expansive intersomatic cage in the expanded position.
Figure 15 shows an isometric perspective of the transforaminal intersomatic cage to which the invention refers.
Figure 16 shows the top, plan and side views of the cage shown in the previous figure.
Figure 17 represents a side view of the final assembly of a transforaminal intersomatic cage of the invention in the intersomatic canal.
Figure 18 shows a cross-sectional plan view of the introduction instrument of the invention, having a transforaminal implant attached thereto.
Figure 19 is an isometric perspective view of the introduction path for the transforaminal implant.

### Preferred Embodiment of the Invention

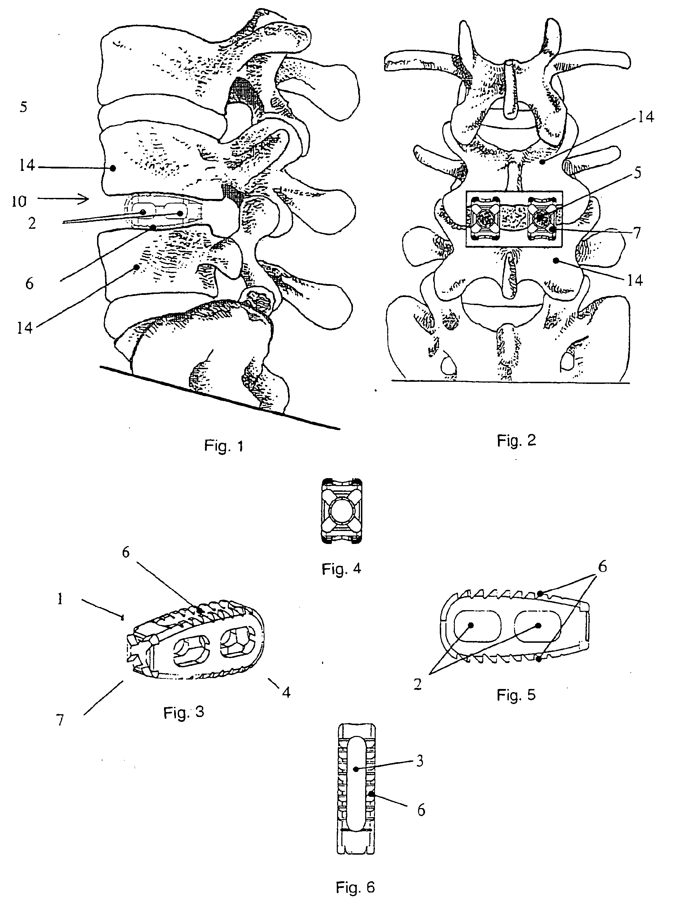

As seen in the attached figures (Figures 1 through 6) the monoblock intersomatic cage 1 is received between two lumbar vertebrae 14 (Figures 1 and 2). The PLIF technique provides for the symmetrical and parallel placement of two cages 1 in each intersomatic space.

This cage comprises side windows 2 and a vertical pass-through window 3, these windows forming the recess 4 in which the bone graft 5 will be received for fusion, or arthrodesis. It is possible to obtain such graft from the patient himself/herself or from a bone bank, or to use a synthetic material. The cage 1 is provided with serrated teeth 6 at both its upper and lower portions, aiming at enhancing the supporting capacity and bearing surface between adjacent vertebrae 14. It is also provided with a coupling system 7 on its back part to introduce the implant by means of the suitable instrument. The sectional geometrical design is essentially oval shaped, so that it can conform the anatomy of the vertebral subcondral disks 14 and keep the natural lordosis or angulation of the spinal column at lumbar level. The correct size will be selected according to the conditions of each case, all cages having identical geometrical characteristics so as to fit any intervertebral size. In addition they will be based on anthropometric studies.

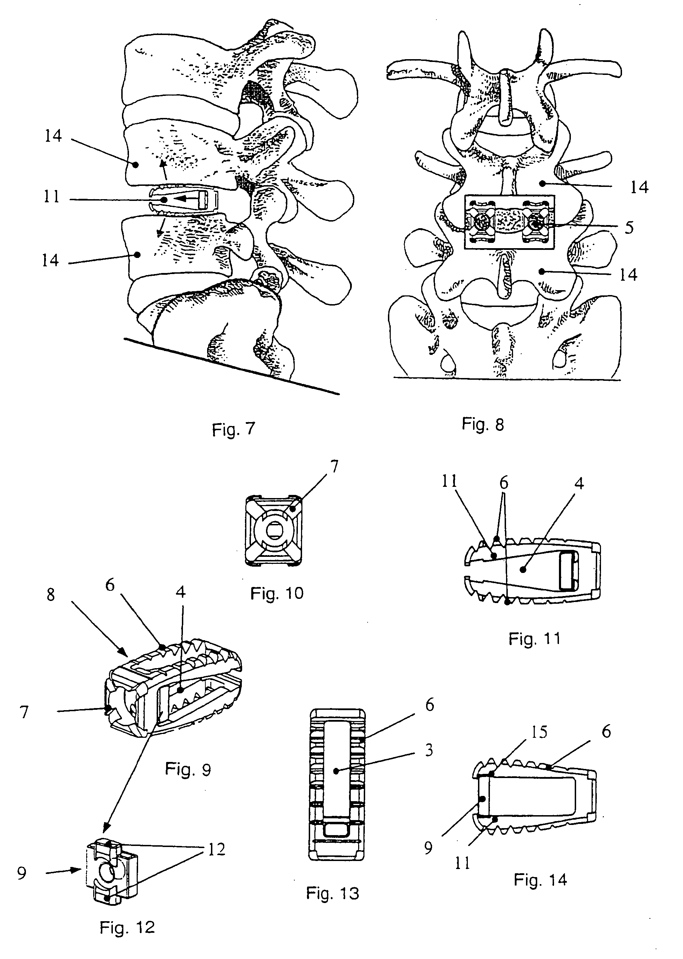

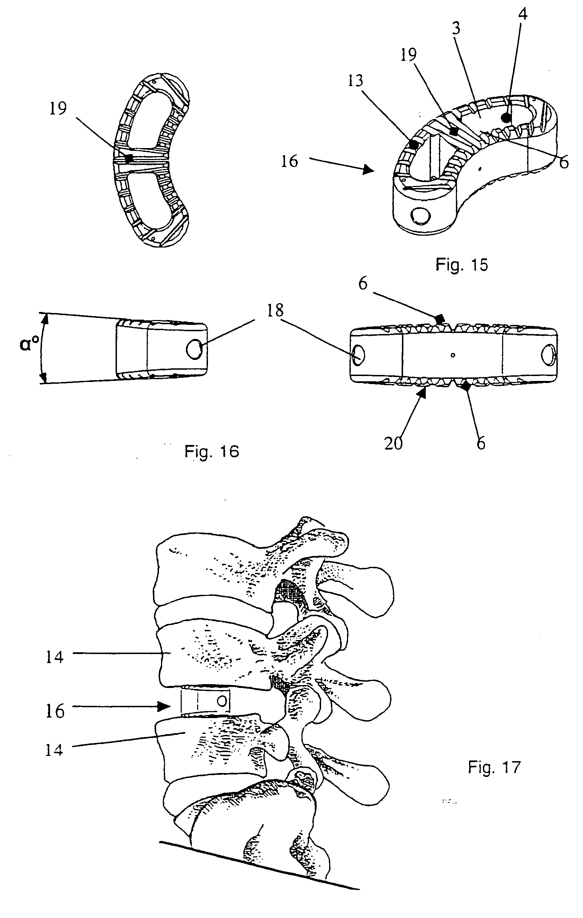

Figures 7 through 17 show the geometrical characteristics of the expansive intersomatic cage 8 and its situation in the surgical field. Like in the case of the single block intersomatic cage, two symmetrical cages are placed in parallel in the intersomatic space existing between two lumbar vertebrae 14 (Figures 7 and 8).

This element comprises a cage 8 that is open at its front part, and an internal washer 9 previously mounted in the inner part of the cage, the washer being able to be slid along an inner canal 4 of the cage, which will be provided with a suitable slope and able to expand the cage 8 at the open or front part, by sliding until it encounters a butt 15 of the cage. At the same time, the washer 9 is guided by some salients or ridges 12 located on another vertical window 3 arranged in the cage 8. The washer sliding motion 9 is made by the suitable instruments once the cage has been introduced in its final position. This cage of the invention is also provided with a few serrated teeth 6 on the upper and lower surfaces of the cage 8 serving the same purpose as that of the monoblock cage. At the same time, there is provided a back coupling system 7 to introduce the cage 8 next to the washer 9 together.

That is, when this cage 8 is at its standing position (Figure 11), the height of the open end is smaller than that of the closed end. However, when the washer 9 is made to slide at the open end on its butts 15, the heights are inverted, being the height of the open end with the washer 9 larger than the height of the cage closed end 8. In any case, the height of the open end is larger when the washer 9 is present at this end, than when said washer is in the opposite end, thus modifying the cage angle.

The application and functioning are very simple. For that purpose, upon identifying the vertebrae 14 to be intervened by applying the corresponding technique, the intersomatic cage is placed between both vertebrae, with their recess 4 being filled with bone material 5. When introducing the cage, the coupling system 7 will be employed as to have the serrated teeth 6 or harpoon make the introduction - but not involuntary removal thereof - easier, as these teeth tend to stick into the subcondral bone in the presence of removal motion, thus enhancing the prosthesis fixation by avoiding expulsion thereof.

As the single block intersomatic cage 1 is introduced in its corresponding place, and because of its angles and oval design, it separates the vertebrae 14 between which it should be placed, so as to follow the general vertebral anatomy, improve the contact surface and therefore, the distribution of the implant loads. In addition, it keeps the natural angulation or lordosis of the spinal cord at these lumbar levels.

The contact between the bone graft 5 and the body of the adjacent vertebrae 14 is attained by the side 2 and upper 3 windows, which helps the osteointegration thus enhancing prosthesis fixation and avoiding failures. At the same time and thanks to these windows on the body 1 and in certain cases, because of the material used for these cages, e.g. the PEEK, examination of the implant is made easier by means of radiographic techniques or the like. Since they are not radio-opaque materials, visibility is possible to better control its evolution.

On the other hand, the variant of the expansive intersomatic cage has some features of its own in terms of structure, as well as some advantages, too. The application method of this cage is the same as that of the monoblock cage. In order to place it, the washer 9 is located in its standing position (Figure 11), so that the cage body inclination 8 facilitates introduction in the intervertebral space. Upon completion, this washer 9 is moved into its working position by applying the corresponding method (Figure 14).

The washer 9 is moved along the interior recess 4, the window 3 serving as a guide, since the salients or ridges 12 are located in the inner part so that sliding along this window is allowed with no transversal movements hindering such sliding motion. At the end of this path, there is a stop 15 against which the washer stays at its working position, thus avoiding back collapse of the cage and the expulsion of the washer itself.

In this situation, the cage body 8 has changed the slope of its inclination by inverting it, so that the spinal cord angulation or lordosis is achieved once the washer 9 has been moved into its final position. This cage is also provided with serrated teeth 6 which, like the at the monoblock cage, prevent involuntary removal because they stick into the vertebra body 14. It is also provided with a cavity 4 for the insertion of bone material 5, and a window 3 which makes the bone fusion with adjacent vertebrae easier.

The transforaminal intersomatic cage 16 is a cage having the shape of a circumference arch, hollow sides, and holes or threads 18 on its ends so as to facilitate introduction thereof. It is provided with a central column 19 to enhance cage strength.

Seen from its side, it its usual working position, it consists geometrically of a wedge or trapezium with two angled faces and two parallel faces that keep a fix lordosis angle (α) in the lumbar vertebrae upon introduction thereof in the patient. At the same time, frontally, in its normal working situation, it is provided with a convex zone 20 domed at its upper and lower parts for anatomical adjustment to a vertebral body 14.

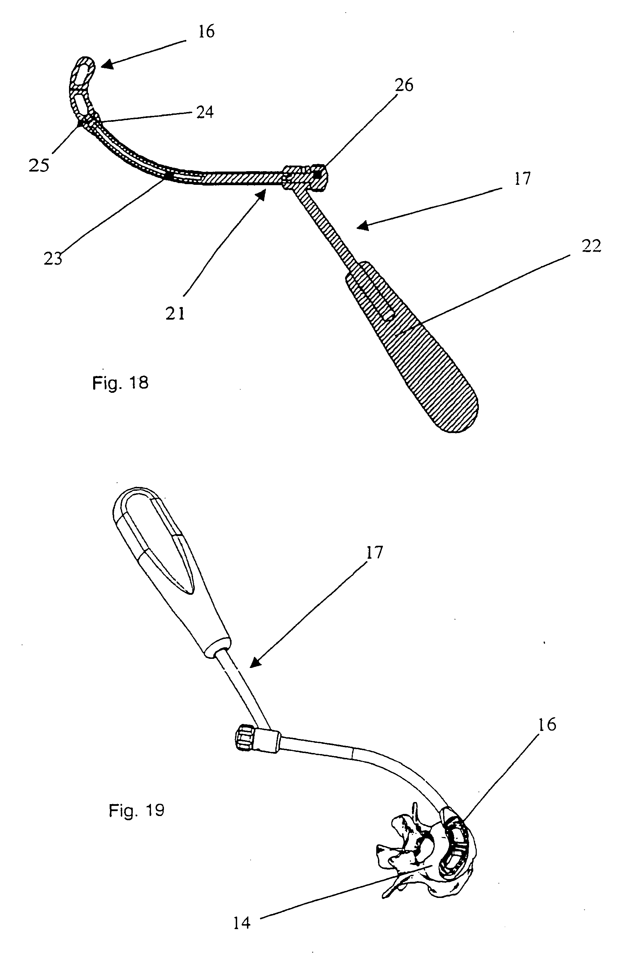

At the same time, the introduction instrument 17 comprises a tube 21 bent at its end, having a handle 22 arranged as depicted in Figures 18 and 19. Inside the tube there is a flexible bar 23 threaded at the curved end 24 thereof, which will serve the purpose of supporting the implant 16. In addition, the instrument is provided of a concave surface 25 that matches the implant 16 so as to avoid rotation. At the other end of the tube 21 there is a torsion device or pommel 26 to transmit the torque and which allows attachment to the implant.

This pommel is integrally to the flexible bar 23 so that when one tries to attach the implant 16 to the instrument 17, said implant should be placed making the hole 18 face the end of the flexible bar 23. Thus a preferably threaded attachment is obtained, in which by rotation of the pommel 26 that is integral to the bar, the implant 16 is also threaded and attached to the instrument 17.

Subsequently, all there is to do is placing this implant in its location via transforaminal, between the vertebrae 14 and disengage the implant from the introduction instrument. In order to achieve this, the pommel 26 is being rotated in the opposite direction, thus releasing said implant. The fact that the end 25 of the tube 21 is concave, matching the implant, prevents this from rotating on itself.

Now that the nature of this invention and a way of putting it into practice have been described, we should add that changes can be made to the shape, materials and arrangement of the invention and its components as long as these alterations do not substantially change the characteristics of the invention described as follows.

In one aspect, the invention relates to an intersomatic cage for posterior fusion surgery to the lumbar column of the type that uses preferably intersomatic cages (1, 8) in order to restore the height of the intersomatic gap between the lumbar vertebras (14), characterized in that the cage comprises lateral windows (2) that allow radiological control of bone growth and one vertical trough (3) that form the hollow inner recess (4) where the bone implant (5) is lodged for the fusion or arthrodesis, an implant that can come from the patient himself, from the bone bank, or can be made from synthetic material; while the cage (1, 8) has some saw-shaped teeth (6) on its top and bottom side intended to increase the holding ability and the support surface between the adjacent vertebras (14); in addition, a coupling system (7) is available at its back side to introduce the implant using the appropriate instrumentation, the geometrical design is of an essentially oval profile in order to couple with the general anatomy improving the contact area, the load distribution in the implant, improving bone fusion and maintaining itself attached to the vertebras (14) by the means of saw-shaped or harpoon-shaped teeth (6) that prevent the expulsion of the implant.

A preferred embodiment of the invention relates to an intersomatic cage for posterior fusion surgery to the lumbar column according to the above, characterized in that the global profile of the cage is angulated, maintaining the lordosis or the natural angulations of the intervened lumbar gaps.

A further preferred embodiment of the invention relates to an intersomatic cage for posterior fusion surgery to the lumbar column according to the above, characterized in that the cage (8) has a lateral angulated guide (11) and another vertical one (3), through which an internal washer (9) is slided along.

Another preferred embodiment of the invention relates to an intersomatic cage for posterior fusion surgery to the lumbar column according to the above, characterized in that the expansive intersomatic cage (8) has a protrusion or step (16) for the fixation of the washer (9) inside the cage.

Another preferred embodiment of the invention relates to an intersomatic cage for posterior fusion surgery to the lumbar column according to the above, characterized in that the washer (9) has some salients (12) that remain lodged inside the trough (3), in a way that it will allow sliding along this trough serving as a guide.

Another preferred embodiment of the invention relates to an intersomatic cage for posterior fusion surgery to the lumbar column according to the above, characterized in that the angulation of the lateral guide (11) is such that the cage (8) adopts a slope when the washer (9) is in its initial or rest position, changing this slope when the washer is in its working position.

Another preferred embodiment of the invention relates to an intersomatic cage for posterior fusion surgery to the lumbar column according to the above,, characterized in that the expansive intersomatic cage (8), with the washer (9) located in its working position on its protrusions (15), falls upon the slope in the vertical direction.

Another preferred embodiment of the invention relates to an intersomatic cage for posterior fusion surgery to the lumbar column according to the above, characterized in that the expansive intersomatic cage (8), with washer (9) located in its working position on its protrusions (15), modifies the slope of the cage (8) in the vertical direction of widening, wherein the height of the open end is larger when the washer (9) is at this end than when it is on the opposite end.

In another aspect, the invention relates to an intersomatic cage for posterior fusion surgery to the lumbar column of the type that preferable uses transforaminal cages (16) in order to restore the intersomatic gap between lumbar vertebras (14), characterized in that the cage (16) comprises a cage shaped as a circumferential arc, hollow (4) at both its lateral parts in order to lodge the bone implant (5), and orifices or threads (18) at its ends to facilitate its introduction; said cage having a central column (19) to give the cage greater rigidity; which cage, from a lateral view, at its usual working position, has a geometrical shape of a wedge or a trapezium with two angulated sides and two parallel sides that maintain a fixed angle of the lordosis (α) at the lumbar vertebras once introduced into the patient.

A preferred embodiment of the invention described above relates to an intersomatic cage for posterior fusion surgery to the lumbar column according to the above, characterized in that, viewing this cage (16) frontally in its usual working position, it has a convex zone (20) in a dome shape at its top and bottom parts for anatomic adaptation to the vertebral body (14).

Another preferred embodiment of the invention relates to an intersomatic cage for posterior fusion surgery to the lumbar column according to the above, characterized in that the transforaminal cage (16) has saw shaped teeth or harpoons (6) at its top and bottom parts.

Described as another aspect of the invention is also a transforaminal implant introducer of the type that is used as a surgical instrument for the implant of intersomatic cages (16) on a transforaminal route, characterized in that the introducer (17) comprises a tube (21) bended at its end, with a handle (22); inside the tube there is a flexible rod (23), threaded at its curved end (24), which will be used to hold the implant (16), and the instrument has a concave surface (25) at this same end, and at the other end of the tube (21) there is a torsion device or pommel (26) for the transmission of the torque that enables its attachment to the implant, this handle being integral part of the flexible rod (23).

In a preferred embodiment, the invention also relates to a transforaminal implant introducer according to the above, characterized in that the concave surface (25) is congruent with the implant (16) to prevent its rotation.

## Claims

1. An intersomatic cage for posterior fusion surgery to the lumbar column of the type that preferable uses transforaminal cages (16) in order to restore the intersomatic gap between lumbar vertebras (14), wherein the cage (16) comprises a cage shaped as a circumferential arc which is hollow (4) at both its lateral parts, said hollow parts being adapted to receive a bone implant (5), said cage (16) having orifices or threads (18) at its ends to facilitate its introduction; and having a central column (19) to give the cage greater rigidity; said cage (16), from a lateral view, at its usual working position, having a geometrical shape of a wedge or a trapezium with two angulated sides and two parallel sides that maintain a fixed angle of the lordosis (α) at the lumbar vertebras once introduced into the patient.

2. The intersomatic cage according to claim 1, said cage (16) having saw-shaped teeth or harpoon-shaped teeth (6), preferably having saw-shaped or harpoone-shaped teeth (6) at its top and bottom parts, only.

3. The intersomatic cage according to claim 1 or claim 2, wherein, viewing the cage (16) frontally in its usual working position, it has a convex zone (20) in a dome shape at its top and bottom parts for anatomic adaptation to the vertebral body (14).

4. The intersomatic cage according to any of the claims 1 to 3, wherein said cavity or hollow parts are adapted to receive a bone implant (5) which may come from the patient himself, may come from a bone bank or may be made of a synthetic material.
